# EUROPEAN PATENT SPECIFICATION

(11) **EP 2 530 465 B1**
(45) Date of publication and mention of the grant of the patent: **29.07.2015**
(21) Application number: 11734623.9
(22) Date of filing: 18.01.2011
(51) Int. Cl.: G01N 33/53, G01N 33/533, G01N 33/534, G01N 33/535, G01N 33/574

(54) **METHOD FOR IMMUNOASSAY OF AUTOANTIBODY AGAINST KU86, KIT FOR USE IN SAME, AND METHOD FOR DETERMINATION OF PRIMARY HEPATOCELLULAR CARCINOMA USING SAME**
VERFAHREN FÜR EIN IMMUNOASSAY MIT AUTOANTIKÖRPERN GEGEN KU86, KIT ZUR VERWENDUNG DARIN SOWIE VERFAHREN ZUR BESTIMMUNG PRIMÄRER BLUTZELLENKARZINOME DAMIT
MÉTHODE POUR L'IMMUNOESSAI D'UN AUTO-ANTICORPS CONTRE KU86, KIT DESTINÉ À ÊTRE UTILISÉ DANS CELLE-CI, ET MÉTHODE POUR LA DÉTERMINATION D'UN CARCINOME HÉPATOCELLULAIRE PRIMAIRE UTILISANT CELLE-CI

(30) Priority: 25.01.2010 JP 2010012976
(43) Date of publication of application: 05.12.2012
(73) Proprietor: Nitto Boseki Co., Ltd, Fukushima-shi, Fukushima 960-8161 (JP)
(72) Inventor: KOJIMA Ryo, Koriyama-shi Fukushima 963-8061 (JP); NODA Kenta, Koriyama-shi Fukushima 963-8061 (JP); SEIMIYA Masanori, Chiba-shi Chiba 260-8670 (JP); SOGAWA Kazuyuki, Chiba-shi Chiba 260-8670 (JP); NOMURA Fumio, Chiba-shi Chiba 260-8670 (JP)
(74) Representative: Hoffmann Eitle
(86) International application number: PCT/JP2011/050720
(87) International publication number: WO 2011/090017

(56) References cited:
- EP-A1- 2 535 715
- FUMIO NOMURA ET AL: "Serum anti-Ku86 is a potential biomarker for early detection of hepatitis C virus-related hepatocellular carcinoma", BIOCHEMICAL AND BIOPHYSICAL RESEARCH COMMUNICATIONS, vol. 421, no. 4, 1 May 2012 (2012-05-01), pages 837-843, XP055062741, ISSN: 0006-291X, DOI: 10.1016/j.bbrc.2012.04.099
- HWEE TONG TAN ET AL: "Serum autoantibodies as biomarkers for early cancer detection", FEBS JOURNAL, vol. 276, no. 23, 26 December 2009 (2009-12-26), pages 6880-6904, XP055067154, ISSN: 1742-464X, DOI: 10.1111/j.1742-4658.2009.07396.x
- YANEVA M ET AL.: 'Antibodies against Ku protein in sera from patients with autoimmune diseases.' CLIN EXP IMMUNOL vol. 76, no. 3, June 1989, pages 366 - 372, XP008161284
- SEIMIYA M ET AL.: 'Identification of novel immunohistochemical tumor markers for primary hepatocellular carcinoma; clathrin heavy chain and formiminotransferase cyclodeaminase.' HEPATOLOGY vol. 48, no. 2, August 2008, pages 519 - 530, XP008161291
- MIMORI T: 'Clinical significance of anti-Ku autoantibodies--a serologic marker of overlap syndrome?' INTERN MED vol. 41, no. 12, December 2002, pages 1096 - 1098, XP008161373
- HIRAKATA M ET AL.: 'Association between autoantibodies to the Ku protein and DPB1.' ARTHRITIS RHEUM vol. 52, no. 2, February 2005, pages 668 - 669, XP008161374

## Description

### TECHNICAL FIELD

The present invention relates to a method for immunoassay for measuring an autoantibody against Ku86. In particular, an autoantibody against Ku86 occurs specifically in the blood of a patient with primary hepatocellular carcinoma. Thus, the present invention not only provides a method of measuring the autoantibody, but also may be utilized for determination of primary hepatocellular carcinoma.

### BACKGROUND ART

Ku86 is a protein which is involved in cleavage of double-stranded DNA, and, together with Ku70, forms a heterodimer, referred to as Ku. The Ku heterodimer has been described to be capable of repairing double-stranded DNA breaks in cooperation with a DNA dependent protein kinase and the like (Non Patent Literature 1).

Meanwhile, it has been revealed that according to a modified agarose two-dimensional electrophoresis in which 2D-DIGE technique (two-dimensional fluorescence difference gel electrophoresis) is applied to agarose two-dimensional electrophoresis (Non Patent Literature 2), by comparing the protein expression levels between a cancerous part of primary hepatocellular carcinoma and a peripheral non-cancerous tissue using proteomic analysis, a protein Ku86 is expressed at a higher level in the cancerous part (Non Patent Literature 3).

HWEE TONG TAN ET AL: "Serum autoantibodies as biomarkers for early cancer detection", FEBS JOURNAL, vol. 276, no. 23, 26 December 2009 (2009-12-26), pages 6880-6904 reviews serum autoantibodies for early cancer detection.

SEIMIYA M ET AL. : "Identification of novel immunohistochemical tumor markers for primary hepatocellular carcinoma; clathrin heavy chain and formiminotransferase cyclodeaminase." HEPATOLOGY vol. 48, no. 2, August 2008, pages 519 - 530 relates to the identification of immunohistochemical tumor markers for primary hepatocellular carcinoma and inter *alia* states that immunoblot confirmed that the expression of Ku86 is increased in tumor.

### CITATION LIST

### Non Patent Literature

Non Patent Literature 1: Li et al., Proc. Natl. Acad. Sci. USA, Vol.9, No.2, 832-837, 2002
Non Patent Literature 2: Takeshi Tomonaga et al., Clin. Cancer Res. 2004;10:2007-2014
Non Patent Literature 3: Masanori Seimiya et al., Hepatology 2008;48:519-30

### SUMMARY OF INVENTION

### TECHNICAL PROBLEM

The present inventors have found that, by measuring an expression level of Ku86 present in a tissue specimen derived from a patient suspected of having carcinoma or a patient with carcinoma, a cancerous part can be distinguished from a non-cancerous part in those tissues. The present inventors have further carried on the study on the presence of Ku86 in a blood specimen, and surprisingly found that an autoantibody against Ku86 may be present in a blood specimen, and the amount of the autoantibody is specifically abundant in a case of a patient with primary hepatocellular carcinoma. Therefore, an object of the present invention is to provide a method of measuring an autoantibody against Ku86, which can be applied to the determination of primary hepatocellular carcinoma.

### SOLUTION TO PROBLEM

The present inventors have found that an autoantibody against Ku86 can be measured by reacting the autoantibody in a specimen with a Ku86 antigen as a reagent, and measuring the resulting immune complex between the autoantibody and the Ku86 antigen with a labeled anti-human immunoglobulin antibody, thereby allowing the determination of primary hepatocellular carcinoma, and then completed the present invention.

Thus, the present invention relates to the following embodiments:
1. A method for determination of primary hepatocellular carcinoma, wherein the method is characterized in that an autoantibody against Ku86 is measured in an immunoassay by reacting the autoantibody against Ku86 in a specimen with a Ku86 antigen as a reagent, and measuring a resulting immune complex between the autoantibody against Ku86 and the Ku86 antigen.
2. The method according to item 1, wherein the specimen is derived from blood.
3. The method according to item 1 or 2, wherein the resulting immune complex is measured by enzyme-linked immunosorbent assay, fluorescence immunoassay, chemiluminescent immunoassay, or radioimmunoassay.
4. A method of determining primary hepatocellular carcinoma, wherein the primary hepatocellular carcinoma is determined by measuring an autoantibody against Ku86.
5. The method of determining primary hepatocellular carcinoma according to item 4, wherein the specimen is derived from blood.
6. Use of a marker for determination of primary hepatocellular carcinoma, wherein the marker comprises an autoantibody against Ku86 and is measured in a specimen.
7. Use of the marker for determination of primary hepatocellular carcinoma according to item 6, wherein the specimen is derived from blood.
8. Marker for use in a method for determining primary hepatocellular carcinoma *in vivo,* wherein the marker comprises an autoantibody against Ku86.

### ADVANTAGEOUS EFFECTS

The present invention can easily measure an autoantibody against Ku86 present in a specimen, in particular, a specimen derived from blood, and it is effective for determination of a patient with primary hepatocellular carcinoma.

### BRIEF DESCRIPTION OF DRAWINGS

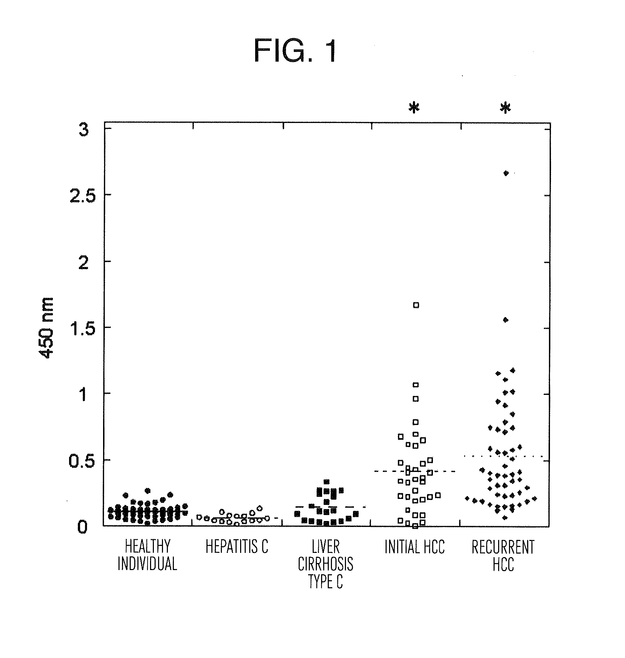

[Fig. 1] Fig. 1 shows results of measurement of an autoantibody against Ku86 in a specimen from a healthy individual, a serum specimen from a patient with hepatitis C, a serum specimen from a patient with liver cirrhosis type C, a serum specimen from a patient with initial primary hepatocellular carcinoma (hereinafter, sometimes referred to as initial HCC) and a serum specimen from a patient with recurrent primary hepatocellular carcinoma (hereinafter, sometimes referred to as recurrent HCC), using an ELISA plate sensitized with a Ku86 antigen. In the figure, the horizontal axis represents disease names, and the vertical axis represents absorbance for light with wavelength of 450 nm. An asterisk represents a group of serum specimen in which there is a significant difference (p < 0.0001) according to Wilcoxon two-sample test, in all cases when compared to the group of serum specimen from healthy individuals, the group of specimen from patients with hepatitis C or the group of serum specimen from patients with liver cirrhosis type C.

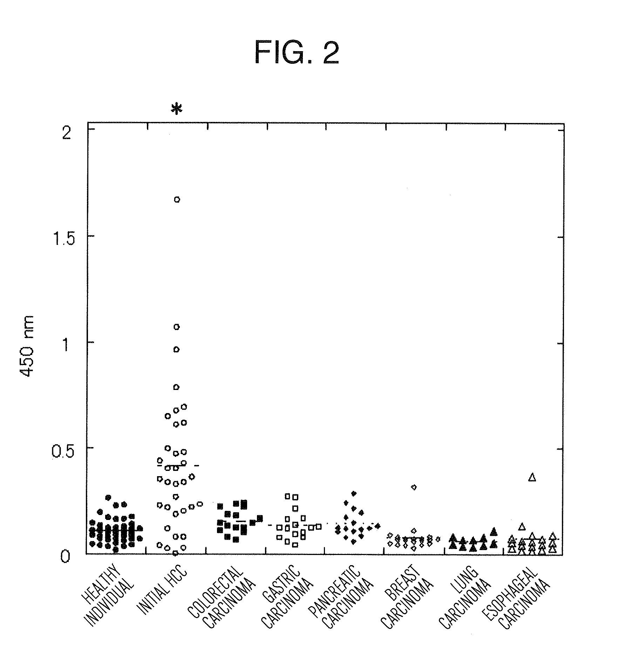

[Fig. 2] Fig. 2 shows results of measurement of an complex between Ku86 and its autoantibody in serum specimen from a healthy individual, serum specimen from a patient with initial HCC, serum specimen from a patient with colorectal carcinoma, serum specimen from a patient with gastric carcinoma, serum specimen from a patient with pancreatic carcinoma, serum specimen from a patient with breast carcinoma, serum specimen from a patient with lung carcinoma, and serum specimen from a patient with esophageal carcinoma, using an ELISA plate sensitized with a Ku86 antigen. In the figure, the horizontal axis represents disease names, and the vertical axis represents absorbance for light with wavelength of 450 nm. An asterisk represents a group of serum specimen in which there is a significant difference (p < 0.0001) according to Wilcoxon two-sample test, in all cases when compared to the group of serum specimen from healthy individuals, the group of serum specimen from patients with colorectal carcinoma, the group of serum specimen from patients with gastric carcinoma, the group of serum specimen from patients with pancreatic carcinoma, the group of serum specimen from patients with breast carcinoma, the group of serum specimen from patients with lung carcinoma, or the group of serum specimen from patients with esophageal carcinoma.

### DESCRIPTION OF EMBODIMENTS

The method for immunoassay of autoantibody against Ku86 of the present invention is used for determination of primary hepatocellular carcinoma and is characterized in that the autoantibody against Ku86 is measured by reacting the autoantibody against Ku86 in a specimen with a Ku86 antigen as a reagent, and measuring the resulting immune complex between the autoantibody against Ku86 and the Ku86 antigen.

In the present invention, a specimen is preferably a sample derived from an organism, in particular, a specimen derived from blood is preferred, and examples of a specimen derived from blood may include whole blood, blood plasma, and serum.

The subject to be measured by the method for immunoassay of the present invention is an autoantibody against Ku86. As described above, Ku86 is a protein which is involved in cleavage of double-stranded DNA, its formal name being ATP-dependent DNA helicase 2 subunit 2. It is otherwise referred to as XRCC5. Additionally, Ku86 is an 82 kDa protein consisting of 732 amino acids, and its accession number (accession No.) in the US National Center for Biotechnology Information (NCBI) is gi-10863945.

To perform the method for immunoassay of the present invention, a Ku86 antigen as a reagent is used. Though a Ku86 antigen as a reagent is not especially limited as long as it may effect an antigen-antibody reaction with an autoantibody against Ku86, examples of it may include a full length Ku86 protein, a variant of the full length Ku86 protein which is a protein having the same function as the full length protein capable of an antigen-antibody reaction with an autoantibody against Ku86, and having a homology of 90% or more to the amino acid sequence, or a protein having the amino acid sequence in which one to several amino acid residues are deleted, substituted, or added in the amino acid sequence of the full length Ku86 protein, and a fragment peptide of Ku86 that may effect an antigen-antibody reaction with the autoantibody against Ku86. Though a full length Ku86 protein is available from Abnova Corporation, the protein or its variant may also be synthesized by genetic engineering technique because its entire amino acid sequence is known as described above. When used in the present invention, a fragment peptide of Ku86 may be generated by cleaving a full length Ku86 protein into various peptide fragments by enzymatic hydrolysis and the like, or may also be easily generated using a commercial automated peptide synthesizer. Additionally, the targeted fragment peptide of Ku86 may be generated by genetic engineering technique.

In the present invention, the thus-obtained variant or fragment peptide of the full length Ku86 protein may be reacted with an autoantibody against Ku86, then those who produce an antigen-antibody reaction may be selected to be used as a Ku86 antigen as a reagent. In the present invention, the whole of the each peptide fragment described above, as well as a part of it and the mixture thereof may be used, and are included in a Ku86 antigen as a reagent.

For the immunoassay of an autoantibody against Ku86 in the present invention, for example, a Ku86 antigen as a reagent is solid-phased onto a microplate or other carrier, and then a specimen expected to contain the autoantibody is applied to the resulting water-insolubilized carrier to allow the autoantibody to be bound thereto by an antigen-antibody reaction with the Ku86 antigen as a reagent. Then, an anti-human immunoglobulin antibody labeled with an enzyme and the like is applied to the carrier to react with and bind to the autoantibody.

The preparation of a water-insolubilized carrier may be easily carried out using a known method that binds a protein to the surface of a solid phase. As a carrier for conversion to solid-phase, for example, beads, microplates, and tubes are generally used. For a method of binding a Ku86 antigen as a reagent to these solid-phase surfaces, known immobilization techniques such as physisorption and chemical binding may be utilized as appropriate.

Upon contacting the thus solid-phased Ku86 antigen with a specimen containing its autoantibody, only the autoantibody against Ku86 binds specifically to the Ku86 antigen as a reagent. Thus, when a labeled anti-human immunoglobulin antibody is added, the anti-human immunoglobulin antibody binds to the autoantibody against Ku86, therefore this label can be utilized to carry out the measurement of the autoantibody.

As a label, routinely used labels, for example, enzymes, radioisotopes, fluorescent and chemiluminescent substances such as FITC, rhodamine, and luminol are used as appropriate. These various labels may be used to measure an autoantibody against Ku86 by a method such as enzyme-linked immunosorbent assay, radioimmunoassay, fluorescence immunoassay, and chemiluminescence immunoassay.

As a labeling enzyme used in enzyme-linked immunosorbent assay, an enzyme that is routinely used in enzyme immunoassay (EIA), such as horseradish peroxidase, calf intestine alkaline phosphatase, β-galactosidase, urease, or glucose oxidase is used as appropriate, and chromogenic substrates suitable for these enzymes and used routinely in EIA are used as appropriate. As chromogenic substrates, for example, in the case of HRP, 3,3',5,5'-tetramethyl benzidine (TMBZ), TMBZ·HCl, TMBZ·PS, ABTS, o-phenylene diamine, and p-hydroxyphenyl acetic acid are used, in the case of alkaline phosphatase, p-nitrophenyl phosphate and 4-methylumbelliferyl phosphate are used, and in the case of β-galactosidase, o-nitrophenyl-β-D-galactopyranoside, and 4-methylumbelliferyl β-D-galactopyranoside are used.

Also in radioimmunoassay, fluorescence immunoassay, chemiluminescence immunoassay and the like, known labels generally used may be adopted.

In the method for immunoassay of the present invention, other than the above-described methods, an autoantibody against Ku86 may also be measured by western blotting, immunohistochemistry, immunoassay such as latex immunonephelometry and immunoprecipitation, and liquid chromatography.

The method for immunoassay of the present invention may be performed by a kit for immunoassay of an autoantibody against Ku86 comprising at least a Ku86 antigen as a reagent component. The Ku86 antigen may be a reagent component of the kit, for example, in the form bound to a water-insoluble carrier such as a microplate. As other reagent components of the kit, an anti-human immunoglobulin antibody is included. For this anti-human immunoglobulin antibody, those labeled with labeling substances such as enzymes, radioisotopes, fluorescent substances, or chemiluminescent substances are used depending on the measuring method adopted, such as enzyme-linked immunosorbent assay, radioimmunoassay, fluorescence immunoassay, and chemiluminescent immunoassay. As other reagent components, a surfactant and a buffer may be added as appropriate.

In the present invention, primary hepatocellular carcinoma can be determined by measuring an autoantibody against Ku86. Measuring an autoantibody against Ku86 by the method of measuring of the present invention is effective for distinguishing a cancerous disease in a patient. Utilization of the method of measuring of the present invention is effective for distinguishing, for example, a patient with initial primary hepatocellular carcinoma and a patient with recurrent primary hepatocellular carcinoma from a healthy individual. Additionally, by utilizing the method of measuring of the present invention, it is possible to distinguish a patient with initial primary hepatocellular carcinoma and a patient with recurrent primary hepatocellular carcinoma from a patient with carcinoma such as a patient with colorectal carcinoma, a patient with gastric carcinoma, a patient with pancreas, a patient with breast carcinoma, a patient with lung carcinoma or a patient with esophageal carcinoma. Furthermore, by utilizing the method of measuring of the present invention, it is possible to distinguish a patient with primary hepatocellular carcinoma such as a patient with initial primary hepatocellular carcinoma or a patient with recurrent primary hepatocellular carcinoma from a patient with hepatitis C or a patient with liver disease such as liver cirrhosis type C.

As apparent from the description above, an autoantibody against Ku86 serves as a marker for determination of primary hepatocellular carcinoma, and may be used as the marker for determination of primary hepatocellular carcinoma. An autoantibody against Ku86 is also preferred as a marker for determining primary hepatocellular carcinoma using a specimen derived from blood such as whole blood, blood plasma, and serum.

### EXAMPLES

Hereinafter, the present invention will be described in more detail with reference to Examples, but the present invention is not to be limited to these Examples in any way.

### Example 1

### Measurement of an autoantibody against Ku86

For serum specimens collected from a healthy individual, a patient with hepatitis C, a patient with liver cirrhosis type C, a patient with initial primary hepatocellular carcinoma, a patient with recurrent primary hepatocellular carcinoma, a patient with colorectal carcinoma, a patient with gastric carcinoma, a patient with pancreatic carcinoma, a patient with breast carcinoma, a patient with lung carcinoma, and a patient with esophageal carcinoma, an autoantibody against Ku86 was measured with enzyme-linked immunosorbent assay (ELISA method) which is specifically described below.

### 1. Method

### (1) Generation of a Ku86-bound ELISA plate

Using an ELISA plate (manufactured by Nalge Nunc International, Maxisorp) as a water-insoluble carrier, full length XRCC5 (recombinant protein GST-tagged: manufactured by Abnova Corporation, 5 µg/mL, 100 µL/well) as Ku86 was placed standstill onto the plate overnight at 4°C to sensitize it, followed by washing the plate with PBS containing 0.05% Tween 20 (200 µL/well) three times. The plate was then coated overnight with PBS containing 1.5% BSA and 10% saccharose (200 µL/well) to generate a Ku86-bound ELISA plate.

### (2) Measurement of an autoantibody against Ku86

Each sample serum was diluted 100 times with PBS, the dilution was added to the Ku86-bound ELISA plate in an amount of 100 µL/well, and placed standstill for 1 hour at 37°C, and the plate was then washed with PBS containing 0.05% Tween 20 (200 µL/well) three times. To the plate, an HRP labeled immunoglobulin (an HRP-labeled anti-Human IgG (manufactured by Zymed Laboratories Inc.) diluted 4000 times with PBS containing 0.05% Tween 20) was added in an amount of 100 µL/well, and the mixture was placed standstill for 30 minutes at 37°C. Then, after washing the plate with PBS containing 0.05% Tween 20 (200 µL/well) three times, TMBZ was added to the plate in an amount of 100 µL/well. After placing the plate standstill for 10 minutes at room temperature, 100 µL/well of 1 N sulfuric acid as a quencher was added to the plate. Absorbance was measured using a microplate reader (manufactured by Bio-Rad Laboratories, Inc.) at the wavelength of 450 nm.

It is noted that specimens from 48 healthy individuals, 16 hepatitis C patients, 21 liver cirrhosis type C patients, 35 specimens from initial primary hepatocellular carcinoma patients, 52 recurrent primary hepatocellular carcinoma patients, 16 specimens from colorectal carcinoma patients, 16 gastric carcinoma patients, 16 pancreatic carcinoma patients, 20 breast carcinoma patients, 10 lung carcinoma patients, and 18 esophageal carcinoma patients were used.

### 2. Results

Results of the measurement of an autoantibody against Ku86 using the Ku86-bound ELISA plate are shown in Fig. 1. For significant difference test, KaleidaGraph 4.0 was used, and statistical processing was carried out with Wilcoxon two-sample test.

As shown in Fig. 1, as compared to the group of healthy individuals, the group of hepatitis C and the group of liver cirrhosis type C, obvious significant differences in the amounts of the autoantibody against Ku86 were observed in the group of specimen from patients with initial primary hepatocellular carcinoma and the group of specimen from patients with recurrent primary hepatocellular carcinoma. Therefore, immunoassay of the autoantibody against Ku86 in blood was shown to be effective for distinguishing primary hepatocellular carcinoma among liver diseases.

As shown in Fig. 2, as compared to the group of healthy individuals and the groups of specimens from patients with various carcinoma, i.e., the group of specimens from patients with colorectal carcinoma, the group of specimens from patients with gastric carcinoma, the group of specimens from patients with pancreatic carcinoma, the group of specimens from patients with breast carcinoma, the group of specimens from patients with lung carcinoma and the group of specimens from patients with esophageal carcinoma, obvious significant difference in the amounts of the autoantibody against Ku86 was observed in the group of specimens from patients with primary hepatocyte. Therefore, immunoassay of the autoantibody against Ku86 in blood was shown to be effective for distinguishing a patient with primary hepatocellular carcinoma from a healthy individual. Furthermore, immunoassay of the autoantibody against Ku86 in blood was shown to be effective for distinguishing a patient with primary hepatocellular carcinoma from a patient with carcinoma such as a patient with colorectal carcinoma, a patient with gastric carcinoma, a patient with pancreas, a patient with breast carcinoma, a patient with lung carcinoma or a patient with esophageal carcinoma.

### INDUSTRIAL APPLICABILITY

As described in detail hereinbefore, an autoantibody against Ku86 can be measured by reacting the autoantibody in a specimen such as a specimen derived from blood with a Ku86 antigen as a reagent, and measuring the resulting immune complex between the autoantibody and the Ku86 antigen, thereby allowing determination of primary hepatocellular carcinoma.

## Claims

1. A method for determination of primary hepatocellular carcinoma, wherein the method is **characterized in that** an autoantibody against Ku86 is measured in an immunoassay by reacting the autoantibody against Ku86 in a specimen with a Ku86 antigen as a reagent, and measuring a resulting immune complex between the autoantibody against Ku86 and the Ku86 antigen.

2. The method according to claim 1, wherein the specimen is derived from blood.

3. The method according to claim 1 or 2, wherein the resulting immune complex is measured by enzyme-linked immunosorbent assay, fluorescence immunoassay, chemiluminescent immunoassay, or radioimmunoassay.

4. A method of determining primary hepatocellular carcinoma, wherein the primary hepatocellular carcinoma is determined by measuring in a specimen an autoantibody against Ku86.

5. The method of determining primary hepatocellular carcinoma according to claim 4, wherein the specimen is derived from blood.

6. Use of a marker for determination of primary hepatocellular carcinoma, wherein the marker comprises an autoantibody against Ku86 and is measured in a specimen.

7. Use of the marker for determination of primary hepatocellular carcinoma according to claim 6, wherein the specimen is derived from blood.

8. Marker for use in a method for determining primary hepatocellular carcinoma *in vivo,* wherein the marker comprises an autoantibody against Ku86.

## Patentansprüche

1. Verfahren zur Bestimmung von primärem hepatozellulären Karzinom, wobei das Verfahren dadurch charakterisiert ist, dass ein Autoantikörper gegen Ku86 im einem Immunassay durch Reagieren des Autoantikörpers gegen Ku86 in einer Probe mit einem Ku86-Antigen als ein Reagenz gemessen wird, und Messen eines resultierenden Immunkomplexes zwischen dem Autoantikörpers gegen Ku86 und dem Ku86-Antigen.

2. Verfahren gemäß Anspruch 1, wobei die Probe von Blut entstammt.

3. Verfahren gemäß Anspruch 1 oder 2, wobei der resultierende Immunkomplex durch Enzyme-Linked-Immunosorbent-Assay, Fluoreszenz-Immunoassay, Chemiluminiszenz-Immunoassay, oder Radioimmunassay gemessen wird.

4. Verfahren zum Bestimmen von primärem hepatozellulären Karzinom, wobei das primäre hepatozelluläre Karzinom bestimmt wird durch Messen, in einer Probe, eines Autoantikörpers gegen Ku86.

5. Verfahren zum Bestimmen von primärem hepatozellulären Karzinom gemäß Anspruch 4, wobei die Probe von Blut entstammt.

6. Verwendung eines Markers zur Bestimmung von primärem hepatozellulären Karzinom, wobei der Marker einen Autoantikörper gegen Ku86 umfasst und in einer Probe gemessen wird.

7. Verwendung des Markers zur Bestimmung von primärem hepatozellulären Karzinom gemäß Anspruch 6, wobei die Probe von Blut entstammt.

8. Marker zur Verwendung in einem Verfahren zum Bestimmen von primärem hepatozellulären Karzinom *in vivo,* wobei der Marker einen Autoantikörper gegen Ku86 umfasst.

## Revendications

1. Procédé de détermination d'un carcinome hépatocellulaire primaire, où le procédé est **caractérisé en ce qu'**un auto-anticorps dirigé contre Ku86 est mesuré dans un dosage immunologique en faisant réagir l'auto-anticorps dirigé contre Ku86 dans un échantillon avec un antigène Ku86 en tant que réactif, et en mesurant un complexe immun résultant entre l'auto-anticorps dirigé contre Ku86 et l'antigène Ku86.

2. Procédé selon la revendication 1, dans lequel l'échantillon est dérivé du sang.

3. Procédé selon la revendication 1 ou la revendication 2, dans lequel le complexe immun résultant est mesuré par un dosage immunoenzymatique, un dosage par immunofluorescence, un dosage immunologique par chimiluminescence ou un dosage radioimmunologique.

4. Procédé de détermination d'un carcinome hépatocellulaire primaire, dans lequel le carcinome hépatocellulaire primaire est déterminé en mesurant, dans un échantillon, un auto-anticorps dirigé contre Ku86.

5. Procédé de détermination d'un carcinome hépatocellulaire primaire selon la revendication 4, dans lequel l'échantillon est dérivé du sang.

6. Utilisation d'un marqueur pour la détermination d'un carcinome hépatocellulaire primaire, où le marqueur comprend un auto-anticorps dirigé contre Ku86 et est mesuré dans un échantillon.

7. Utilisation du marqueur pour la détermination d'un carcinome hépatocellulaire primaire selon la revendication 6, où l'échantillon est dérivé du sang.

8. Marqueur destiné à être utilisé dans un procédé de détermination d'un carcinome hépatocellulaire primaire *in vivo,* où le marqueur comprend un auto-anticorps dirigé contre Ku86.
